# EUROPEAN PATENT SPECIFICATION

(11) **EP 0 506 666 B1**
(45) Date of publication and mention of the grant of the patent: **16.07.1997**
(21) Application number: 90915888.3
(22) Date of filing: 19.10.1990
(51) Int. Cl.: C07H 15/12, C12Q 1/68, C12P 21/06, C12P 21/02, C12P 21/04, C12P 19/34, C12N 15/00, C12N 1/20

(54) **METHOD FOR THE PRODUCTION OF CATALYTIC RNA IN BACTERIA**
VERFAHREN ZUR HERSTELLUNG VON KATALYTISCHER RNS IN BAKTERIEN
PROCEDE DE PRODUCTION D'ARN CATALYTIQUE DANS DES BACTERIES

(43) Date of publication of application: 07.10.1992
(73) Proprietor: CITY OF HOPE, Duarte California 91010-0269 (US)
(72) Inventor: ROSSI, John, J., Glendora, CA 91740 (US); TAYLOR, Nerida, Loma Linda, CA 92354 (US)
(74) Representative: Dowden, Marina
(86) International application number: US9006032
(87) International publication number: WO9206988

(56) References cited:
- EP-A- 0 387 775
- WO-A-91/03162
- JOURNAL OF CELLULAR BIOCHEMISTRY Supplement 13B, 1989; page 289, Abstract G328;
- ROSSI, J.J. ET AL.: 'Ribozymes targeted to HIV-1 RNAs'
- JOURNAL OF CELLULAR BIOCHEMISTRY Supplement 14A, 1990; page 374, Abstract D428; ROSSI, J.J. ET AL.: 'Ribozyme mediated intracellular immunity to HIV-1 in CD4+ HeLa cells'
- Science, Volume 228, No. 4202, issued 24 May 1985 (Washington, DC), M. BAER et al., "A Catalytic RNA and its Gene from Salmonella Typhimurium", see pages 999- 1002.
- Proc. Natl. Acad. Sci., Volume 86, issued December 1989 (US), CAMERON et al., "Specific Gene Suppression by Engineered Ribozymes in Monkey Cells", see pages 9139-9143.
- CHEMICAL ABSTRACTS, Volume 112, No. 7, issued 12 February 1990 (Columbus, Ohio, US), GERLACH et al., "Synthetic Ribozymes for in vivo Inactivation of Prokaryotic or Cukaryotic RNA Transcripts", see page 337, column I, see Abstract No. 51284; Eur. Pat Appl. EP 321,201, 21 June 1989.
- CHEMICAL ABSTRACTS, Volume 110, No. 21, issued 22 May 1989 (Columbus, Ohio, US), CECH et al., "RNA Ribozyme Polymerase, Dephorylase Restriction Endoribonuclease and methods for their manufactors", see page 226, column 2, the Abstract No. 187321K, PCT Int. Appl. WO8804,300, June 1988.
- Proc. Natl. Acad. Sci., Volume 834, issued December 1987 (US), KIM et al., "Three-dimensional Model of the Active Site of the Self-splicing rRNA Precursor of Tetrahymena", see pages 8788-8792.
- EMBO Journal, Volume 8, No. 12, issued 1989, COTTEN et al., "Ribozyme Mediated Destruction of RNA in vivo", see pages 3861-3866.

## Description

This invention relates to a method for the production of catalytic RNA (ribozymes) in cells, including mammalian and bacterial cells. More particularly, the invention relates to synthetic genes which encode a ribozyme, to mammalian and bacterial cells having such genes transformed therein, and to the ribozyme containing expression products of such bacteria. The invention also includes the in vitro and therapeutic use of such expression products.

### BACKGROUND OF THE INVENTION

One form of gene expression impairment by RNA-RNA duplex formation has been termed "antisense" inhibition. Exploitation of antisense gene regulation could lead to potent anti-viral therapy. A serious limitation of the antisense approach, especially as it applies to anti-viral activity, is that it is stoichiometric and may require large molar excesses of anti-sense versus target RNA to be effective.

Within recent years, discoveries of ribozymes, e.g., RNAs with enzymatic activities have led to the development of antisense molecules which not only form RNA-RNA hybrids, but catalytically cleave the covalent phosphodiester linkages and turn over large numbers of substrate molecules. Ribozymes can now be targeted to virtually any RNA transcript, and efficient cleavage can be readily achieved in vitro. See, Kim, S.H., et al. Proc. Natl Acad. Sci. U.S.A. 84:8788-8792 (1987); Haseloff, J., et al., Nature 234:585-591 (1988); PCT published application WO/89/05852; Cech, T.R. JAMA 260:3030-3034 (1988); PCT published application WO/88/04300; Jeffries, A.G., et al., Nucleic Acids Research 17:1371-1377 (1989).

United States Patent 5,144,109 describes stable, catalytically efficient ribozymes useful, inter alia, to cleave HIV-1 RNA or any other viral or endogenous cellular RNA in vitro and in vivo, mammalian cells transformed with such ribozymes, vectors useful to accomplish such transformation and the use for human therapy of such ribozymes whether produced synthetically or as expressed by such transformed cells. See Chang, et al. Clinical Biotechnology 2:23-31 (1990) which is incorporated herein by reference.

EP-A-0 387 775 describes how synthetically produced ribozymes directed to a specific sequence show a cleaving activity even in a medium which contains protein. This application describes a genetic unit which comprises the transcription units necessary for transcription by polymerase III and a DNA coding for RNA-inhibiting RNA, which is arranged in such a way that the inhibiting RNA is a part of a polymerase III transcript. A particularly suitable carrier gene transcribed by polymerase III is tRNA.

Abstract D428 from Journal of Cellular Biochemistry Supplement 14A, 1990, January 13-28, describes the development of a "hammerhead" ribozyme to develop molecules which specifically cleave HIV-1 RNAs. These ribozymes have been transfected into Hela CD4⁺ cells using an expression vector which has the ribozyme under the control of the human β-actin promoter. The structure of such "hammerhead" ribozymes is considered in abstract G328 in the Journal of Cellular Biochemistry, Supplement 13B, 1989, 21st January-11th February.

### SUMMARY OF THE INVENTION

The production of large amounts of specifically targeted ribozymes for therapeutic use is problematical. This invention provides a novel method for the large scale production of any ribozyme by expression of a stable E. coli RNA molecule into which catalytic RNA has been inserted. Any stable E. coli RNA can be utilized.

Hence, the invention relates to a vector comprising a procaryotic promoter system and a ribozyme fused to a stable E. coli RNA gene which comprises from about 50 to 200 nucleotides. The ribozyme is preferably fused to an E. coli 4.5 S RNA gene. The ribozyme is preferably an anti-HIV-1 gag ribozyme.

In the preferred practice of the invention, E. coli is transformed by Tac promoter driven 4.5 S RNA having a catalytic RNA sequence inserted therein. High levels of expression of such fusion RNAs can be achieved by varying the temperature of growth, using mutants of E. Coli or other bacteria which are defective in certain enzymatic activities such as RNAse III, inducing expression with isopropyl thio β galactopyranoside (IPTG) in an appropriate host, preparing a set of nested deletions within the 4.5 S or similar gene to eliminate undesirable folding and by other standard molecular biological techniques.

The invention also includes synthetic genes which encode catalytic RNA, microorganisms transformed with such genes, the catalytic RNA containing expression products of such genes and the in vitro and therapeutic use of such expression products.

### DESCRIPTION OF THE FIGURES

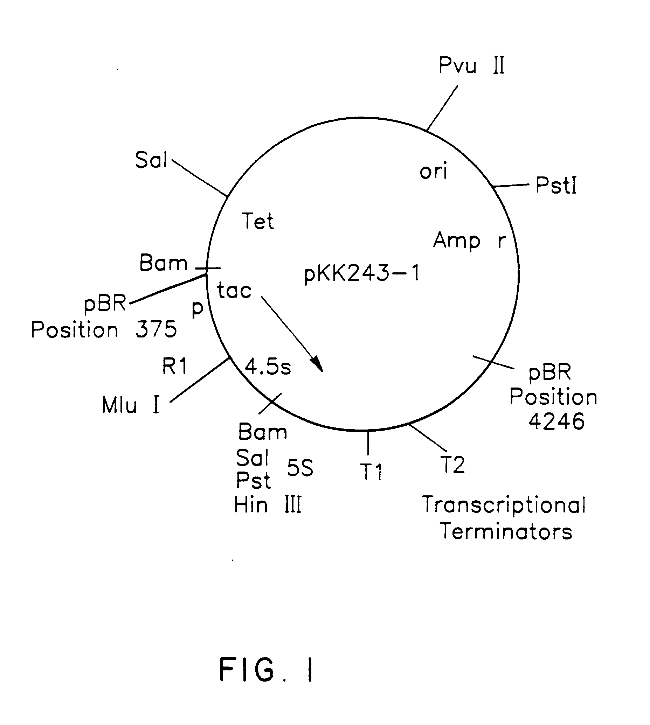

Figure 1 illustrates a vector containing a Tac promoter and an E Coli 4.5 S RNA gene.

Figure 2 illustrates the vector shown by Figure 1 with the transcriptional unit containing the ribozyme.

Figure 3 is a Northern analysis showing the expression of an anti-HIV-1 gag ribozyme fused with 4.5 S RNA (lanes a and b) and 4.5 S RNA alone (lane c).

Figure 4 depicts an ethidium bromide stained gel of an acid-phenol extract of small RNAs overproduced by E. Coli transformants in accordance with the invention.

Figure 5 depicts a preparative gel including ethidium bromide stained RNAs as shown by Figure 4. The arrowhead points to the fusion ribozymes.

Figure 6 is a composite depiction of the RNA folding of the ribozyme embedded in the 4.5 S transcript.

Figure 7 depicts a gel electrophoresis analysis demonstrating in vitro cleavage reactions using the E. Coli transformants produced by 4.5 S fusion ribozymes.

### DETAILED DESCRIPTION OF THE INVENTION

Heretofore, the preferred way to make ribozymes for in vitro studies or delivery to cells in culture was to scale up an in vitro transcription reaction. This invention eliminates the need for in vitro transcription thus greatly reducing the cost of producing large quantities of ribozyme.

In its more generic embodiments, the invention provides a novel method for the large scale production of any ribozyme by the expression of a stable E. coli RNA molecule into which a ribozyme sequence has been inserted.

The stable RNA can be any RNA and the insert can be any ribozyme. More specifically the RNA molecule is small and contains from about 50 to 200 nucleotides and is preferably E. coli 4.5 S RNA.

Among others, any of the ribozymes described in United States patent no. 5,144,109 (application Serial No. 369,489) or Serial No. 401,613 (abandoned) may be used. A strong procaryotic promoter system is chosen to facilitate overproduction of the RNA-ribozyme fusion product. Vectors including the stable RNA, ribozyme and promoter are transformed into bacteria selected to abundantly produce such fusion products.

In general, consensus sequence promoters are useful in the practice of this invention. The Tac promoter system is preferred. The thermally inducible lambda pL promoter system may also be utilized.

Vectors comprising, in combination, a stable E. coli RNA, an inserted ribozyme, and an appropriate procaryotic promoter system and a transcriptional terminator are an important component of the invention. Such vectors are constructed and transformed into bacteria in known manner.

### EXAMPLE I

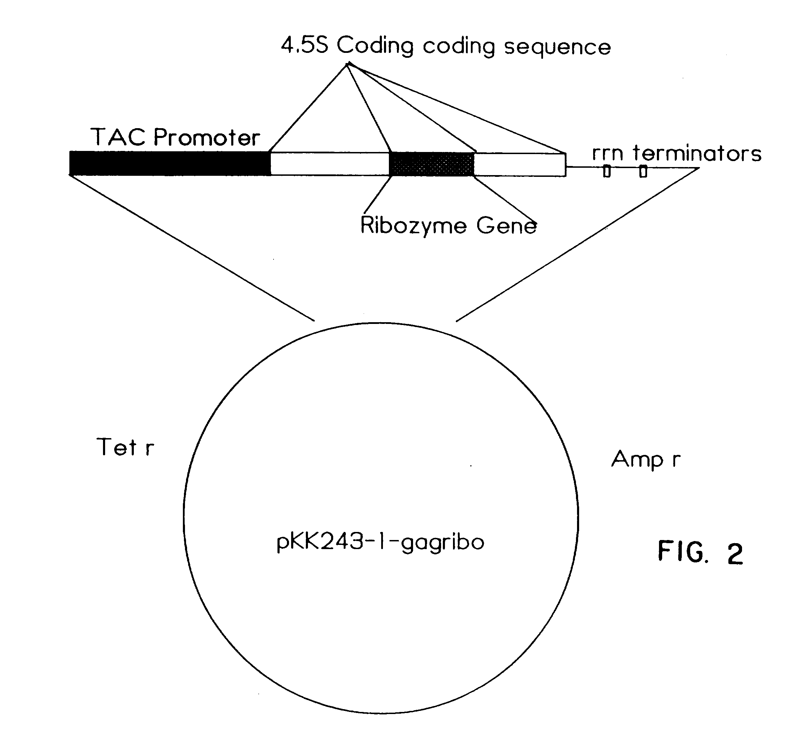

The E. Coli 4.5 S RNA gene is described in the prior art. See Brosius, J., et al., Biological Chemistry 260:3539-3541 (1985). This gene includes an MluI restriction site. The anti-HIV-1 gag ribozyme gene having the sequence 5' GGATCCGCTTAATACTCTGATGAGTCCGTGAGGACGAAACGCT CTCGCACCGGATCC 3' is inserted into the MluI site of the 4.5 S RNA molecule to produce the Figure 2 construct in the following manner:

The plasmid pKK243-1 was cut at the unique MluI restriction site in the 4.5 S gene sequence. The staggered ends created by this digestion were filled in by Klenow DNA polymerase and dephosphorylated with calf intestine phosphatase. Two synthetic DNA oligomers with 10 bases of complementary sequences at their 3' termini, GAGHAM 1(GGCCGGATCCGCTTAATACTCTGATGAGTCCGTGAGGAC) and 2(CCGGATCCGCGAGAGCGTTTCGTCCTCACGG), were polymerized into a double stranded fragment with Taq polymerase by mixing equimolar amounts together in a 50 µliter reaction containing ca. 0.5 µgms of each oligo, 125 µmoles each dNTP, Tag polymerase buffer (Cetus-Perkin Elmer) and 2.5 units of Taq polymerase.

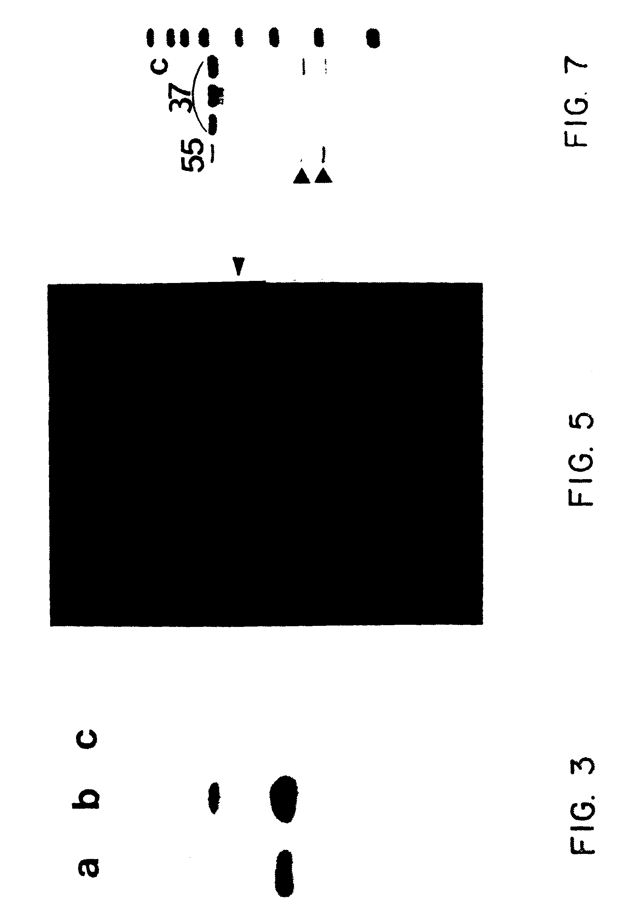

A series of 10 amplification cycles was carried out using the following: 94C-1 min., 37C-1 min. and 72C-2 min. The polymerized fragment was phosphorylated, ligated with pKX243-1 vector and transformed into E. Coli strain MC 1061 by standard calcium chloride techniques. Transformants were screened for the ribozyme insert and clones harboring the insert were examined for expression using a Northern gel analysis (see Figure 3). Low molecular weight RNA was harvested from positive clones by the following method. Cells were suspended in cold 50 mM Tris pH 7.5, 5 mM MgCl₂ and acid phenol extracted twice, made to 0.3 M NaOAc pH 5.5 and precipitated with 3.5 volumes of ethanol. The pellet was washed with 70% ethanol and resuspended in sterile water. Aliquots of this RNA were run on a 6% acrylamide (20:1 acrylamide:bis) gel at 40 millamps in TBE buffer, transferred to Zeta Probe (registered trade mark) nylon filter by electroblotting at 90mamps overnight, and hybridized with radioactively labeled gag ribozymes specific probe GAGHAM 2. Such Northern analysis showed both precursor (lane c) and final products (lanes a and b, Figure 3). The clone with the highest expression was chosen for further study. Both the precursor and processed product were cultured in "L" broth at 37°C and monitored for ribozyme activity after they were purified by overnight diffusion elution from the acrylamide gel in 0.3 M NaOAc pH 5.5, 0.1% SDS. Following elution, the RNA was extracted 2 times with phenol, once with dichloromethane, ethanol precipitated, pelleted by centrifugation and washed with 70% ethanol. The RNA was dissolved in water before use. RNA fragments were tested for in vitro cleavage activity by the methods described by Chang, et al., supra.

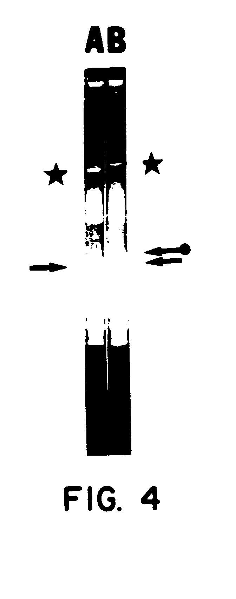

Figure 4 depicts an ethidium bromide stained gel of an acid-phenol extract of small RNAs by which 4.5 S (left lane) and 4.5 S with an 8bp insertion on the MluI site (right lane) which were overproduced by such transformants. The arrows indicate the ethidium bromide stained products (4.5 S) (4.5 S and 8pb insertion) derived from less than 1 ml of cell culture.

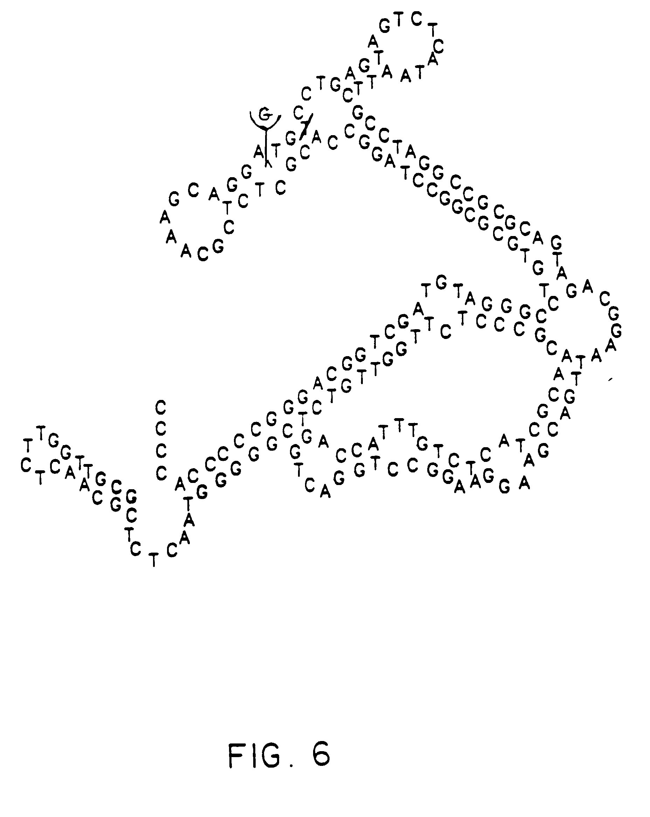

Figure 5 depicts a preparative acid-phenol extract of RNA from approximately 250 mls of cell. The arrow points to the 4.5 S gag ribozyme fusion transcript. This transcript was excised from the gel and shown to be catalytically functional (see Figure 7).

## Claims

1. A vector comprising a procaryotic promoter system and a ribozyme fused to a stable E. coli RNA gene which comprises from about 50 to 200 nucleotides.

2. A vector according to claim 1 wherein said ribozyme is fused to an E. coli 4.5 S RNA gene.

3. A vector according to any of claims 1 and 2 wherein said promoter system is selected from a Tac promoter and a lambda pl promoter.

4. A vector according to any of the preceding claims wherein the ribozyme is an anti-HIV-1 gag ribozyme.

5. A vector as claimed in Claim 4 in which the anti-HIV-1 gag ribozyme is encoded by a gene having the sequence 5'GGATCCGCTTAATACTCTGATGAGTCCGTGAGGACGAAACGCTCTCGCA CCGGATCC 3'.

6. A cell transformed with a vector as claimed in any one of claims 1 to 5.

7. A cell as claimed in Claim 6 which is a bacterial cell.

8. A cell as claimed in Claim 7 which is an E. coli cell or a mutant thereof.

## Patentansprüche

1. Ein ein procaryotisches Promotorsystem und ein zu einem stabilen E.coli-RNA Gen fusioniertes Ribozym enthaltender Vektor, welcher von etwa 50 bis 200 Nucleotide aufweist.

2. Vektor nach Anspruch 1, worin das Ribozym an E.coli-4,5S-RNA Gen fusioniert ist.

3. Vektor nach einem der Ansprüche 1 und 2, worin das Promotorsystem aus einem Tac-Promotor und einem lambda-PL-Promotor ausgewählt ist.

4. Vektor nach einem der vorhergehenden Ansprüche, worin das Ribozym ein Anti-HIV-1gag-Ribozym ist.

5. Vektor nach Anspruch 4, in welchem das Anti-HIV-1gag-Ribozym durch ein Gen mit der Sequenz 5' GGATCCGCTTAATACTCTGATGAGTCCGTGAGG ACGAAACGCTCTCGCACCGGATCC 3' codiert ist.

6. Eine mit einem Vektor nach einem der Ansprüche 1 bis 5 transformierte Zelle.

7. Zelle nach Anspruch 6, welche eine bakterielle Zelle ist.

8. Zelle nach Anspruch 7, welche eine E.coli-Zelle oder eine Mutante davon ist.

## Revendications

1. Vecteur comprenant un système promoteur procaryote et un ribozyme fusionné à un gène d'ARN de E. coli stable qui comprend d'environ 50 à 200 nucléotides.

2. Vecteur selon la revendication 1, dans lequel ledit ribozyme est fusionné à un gène d'ARN de E. coli 4.5 S.

3. Vecteur selon l'une quelconque des revendications 1 et 2, dans lequel ledit système promoteur est choisi parmi un promoteur Tac et un promoteur lambda p1.

4. Vecteur selon l'une quelconque des revendications précédentes, dans lequel le ribozyme est un ribozyme gag anti-VIH-1.

5. Vecteur selon la revendication 4, dans lequel le ribozyme gag anti-VIH-1 est codé par un gène ayant la séquence 5'GGATCCGCTTAATACTCTGATGAGTCCGTGAGGACGAAACGCTCTCGCA CCGGATCC 3'.

6. Cellule transformée par un vecteur selon l'une quelconque des revendications 1 à 5.

7. Cellule selon la revendication 6, qui est une cellule bactérienne.

8. Cellule selon la revendication 7, qui est une cellule de E. coli ou un mutant de cette dernière.
